# EUROPEAN PATENT SPECIFICATION

(11) **EP 0 465 879 B1**
(45) Date of publication and mention of the grant of the patent: **29.11.1995**
(21) Application number: 91109980.2
(22) Date of filing: 18.06.1991
(51) Int. Cl.: C07C 59/88, C07C 69/738, C07C 235/34

(54) **Derivatives of 4-(2,4-difluoro-biphenylyl)-2-methyl-4-oxo-butanoic acid**
Derivate der 4-(2,4-Difluorbiphenyl)-2-methyl-4-oxobutansäure
Dérivés de l'acide 4-(2,4-difluorobiphénylyl)-2-méthyl-4-oxobutanoique

(30) Priority: 18.06.1990 CS 3027/90
(43) Date of publication of application: 15.01.1992
(73) Proprietor: VYZKUMNY USTAV PRO FARMACII A BIOCHEMII STATNI PODNIK, 130 60 Praha 3 (CZ)
(72) Inventor: Kuchar, Miroslav, 130 00 Praha 3 (CS); Poppova, Marie, 130 00 Praha 3 (CS); Grimova, Jaroslava, Dr., 150 00 Praha 5 (CS); Maturová, Eva, Dr., 106 00 Praha 10 (CS)
(74) Representative: von Füner, Alexander, Dr.

(56) References cited:
- CHEMICAL ABSTRACTS, vol. 108, no. 5, February 1, 1988 Columbus, Ohio, USA M.KUCHAR et al. " Metabolic model and QSAR of long-acting anti-inflammatory arylali- phatic acids" page 18, abstract-no 31 304e

## Description

This invention relates to derivatives of 4-(2,4-difluorobiphenylyl)-2-methyl-4-oxobutanoic acid represented by the following general formula (I) 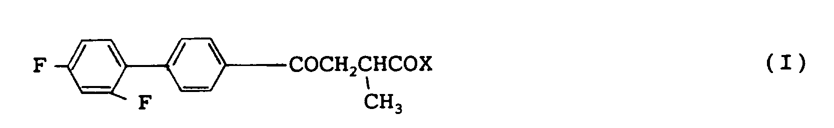
wherein X stands for morpholinyl,
or NHR in which R is an alkyl of 1 to 4 carbon atoms
or a CH₂COOC₂H₅ group,
or OR' in which R' is an alkyl of 1 to 4 carbon atoms,
or O⁻B⁺ group wherein B⁺ is either an alkaline metal cation, alkaline earth cation, cyclohexylamine cation, or lysine cation.

Recently a significant prolonged anti-inflammatory effect of 4-(2,4-difluorobiphenylyl)-2-methyl-4-oxobutanoic acid has been recognized (CS-AO 243 570). An analogous anti-inflammatory effect was found in several functional derivatives and salts of said acid. Some of the substances of general formulae (I) are characterized by physico-chemical properties, incl. increased solubility in water, which extend their therapeutic application without any noticeable decrease of their anti-inflammatory effect. Changes in lipophility eventually differing biotransformation of esters and amides in comparison with the free acid may influence the pharmacokinetic behaviour of these derivatives. Results of the pharmacological evaluation of substances (I) are summarized in Table (I). Simultaneously, these substances are characterized by low toxicity, incl. a low ulcerogenic effect. They may be used for the preparation of therapeutic compositions which may contain the effective substance in combination with pharmacologically acceptable ingredients, liquid or solid, which are usually used in the manufacture of dosage forms.

### Example 1

### Ethoxycarbonylmethylamide 4-(2,4-difluorobiphenylyl)-2-methyl-4-oxobutanoic acid

5.0 g of 4-(2,4-difluorobiphenylyl)-2-methyl-4-oxobutanoic acid is dissolved in a mixture of 35 ml dimethylformamide and 150 ml dichlormethane. 2.1 g N-ethylpiperidine is added to the solution and 2.05 g ethylchloroformiate is added after cooling to -15°C. After stirring for 30 minutes at -15°C the mixture is cooled to -30°C and 1.7 g glycineethylester in 35 ml dichlormethane is added. The mixture is left at room temperature to warm up to 20°C and at this temperature it is stirred for 2 hours. Thereafter, the mixture is consecutively washed 3 times in a separation funnel in 100 ml 5% NaHCO₃, once in 100 ml H₂O, 3 times in 100 ml 1 N HCl, and once again in 100 ml H₂O.

Water is adsorbed by Na₂SO₄ from the organic part which is then dried by evaporation and the crystalline residue is purified by crystallization in acetone. Yield: 4.3 g (67.2 % theor.) of the ethoxycarbonylmethylamide (melting point 95-96°C);

| for C₂₁H₂₁NF₂O₄ (389.4) | | | | |
|---|---|---|---|---|
| calculated: | 64.77 % C, | 5.44 % H, | 3.58 % N, | 9.76 % F; |
| found: | 64.84 % C, | 5.35 % H, | 3.34 % N, | 9.66 % F; |

¹H-NMR (C²HCl₃): δ (2-CH₃) = 1.30 d, J = 7.0 Hz;
δ (NH) = 6.36 bt;
δ (aromat. ortho-CH) = 8.05 d, J = 8.5 Hz.
Similarly were prepared:
Ethylamide of 4-(2,4-difluorobiphenylyl)-2-methyl-4-oxobutanoic acid with a 72.8 % recovery (melting point 128°C, crystallized in acetone);

| for C₁₉H₁₉NF₂O₂ (331.4) | | | | |
|---|---|---|---|---|
| calculated: | 64.77 % C, | 5.44 % H, | 3.58 % N, | 9.76 % F; |
| found: | 64.84 % C, | 5.35 % H, | 3.34 % N, | 9.66 % F; |

¹H-NMR (C²HCl₃): δ (2-CH₃) = 1.30 d, J = 7.0 Hz;
δ (NH) = 6.36 bt;
δ (aromat. ortho-CH) = 8.05 d, J = 8.5 Hz.
Morpholide of 4-(2,4-difluorobiphenylyl)-2-methyl-4-oxobutanoic acid with a 65.8 % recovery (melting point 95-97°C, crystallized in acetone/n-hexane, 4:1);

| for C₂₁H₂₁NF₂O₃ (373.4) | | | | |
|---|---|---|---|---|
| calculated: | 67.55 % C, | 5.69 % H, | 3.75 % N, | 10.18 % F; |
| found: | 67.36 % C, | 5.81 % H, | 3.66 % N, | 10.29 % F; |

¹H-NMR (C²HCl₃): δ (2-CH₃) = 1.22 d, J = 7.0 Hz;
δ (CH₂ morpholine) = 3.68 bs;
δ (aromat. ortho-CH) = 8.07 d, J = 8.5 Hz.

### Example 2

### Ethylester of 4-(2,4-difluorobiphenylyl)-2-methyl-4-oxobutanoic acid

1.2 ml thionylchloride is added to 20 ml ethanol, cooled to - 15°C and then, 4.0 g 4-(2,4-difluorobiphenylyl)-2-methyl-4-oxobutanoic acid is added in four portions while the temperature is kept at -10°C by cooling. At this temperature it is stirred for 30 minutes; then, instead of cooling the mixture is heated to 40-45°C for two hours. Ethanol is vacuum evaporated and the residue is dried by double vacuum distillation with 20 ml benzene. After crystallisation in acetone the crude product gives 3.0 g (68.5 % theor.) of ethylester (melting point 67-68°C)

| for C₁₉H₁₈F₂O₃ (332.4) | | | |
|---|---|---|---|
| calculated: | 68.66 % C, | 5.46 % H, | 11.43 % F; |
| found: | 68.41 % C, | 5.41 % H, | 11.37 % F; |

¹H-NMR (C²HCl₃): δ (2-CH₃) = 1.30 d, J = 7.0 Hz;
δ (CH₂) ester = 4.16 q, J = 7.0 Hz;
δ (arom. ortho-CH) = 8.06, J = 8.5 Hz;
Similarly were prepared:
Isobutylester of 4-(2,4-difluorobiphenylyl)-2-methyl-4-oxobutanoic acid with a 90.5 % recovery (melting point 50-52°C)

| for C₂₁H₂₂F₂O₃ (360.4) | | | |
|---|---|---|---|
| calculated: | 69.98 % C, | 6.15 % H, | 10.54 % F; |
| found: | 69.78 % C, | 6.16 % H, | 10.28 % F; |

¹H-NMR (C²HCl₃): δ (CH₃ isobutyl) = 0.92 d,
J = 7.0 Hz;
δ (2-CH₃) = 1.30 d, J = 7.0 Hz;
δ (aromat.ortho-CH)= = 8.08 d, J = 8.5 Hz.

### Example 3

### n-Butylamide of 4-(2,4-difluorobiphenylyl)-2-methyl-4-oxobutanoic acid

0.2 ml dimethylformamide is added to a suspension of 6.0 g 4-(2,4-difluorobiphenylyl)-2-methyl-4-oxobutanoic acid in 40 ml benzene and then, within 10 minutes 20 ml solution of thionylchloride in 20 ml benzene is added. Then, the mixture is heated to boiling (the suspension becomes a solution) and kept at this temperature while stirring for 45 minutes. The reaction mixture is vacuum condensed and the residue redissolved in 50 ml benzene and condensed until dry. The obtained chloride of 4-(2,4-difluorobiphenylyl)-2-methyl-4-oxobutanoic acid is mixed with 50 ml benzene and the solution is filtered with activated carbon. The clear filtrate is slowly added to a solution of 2.90 g of n-butylamine in 20 ml benzene cooled to 5°C. The mixture is then kept for 1 hour at 5°C and for 2 hours at 20°C. The opaque solution is washed consecutively in 100 ml 1N HCl, 100 ml H₂O, twice in 100 ml 5 % NaHCO₃ and again in 100 ml H₂O. After drying with magnesiumsulphate the benzene is distilled off in vacuum and the crude product is crystallized in acetone. n-Butylamide of 4-(2,4-difluorobiphenylyl)-2-methyl-4-oxobutanoic acid (melting point 114-115°C) in recovery of 72.5 % is obtained.

| for C₂₁H₂₃F₂O₃ (359.4) | | | | |
|---|---|---|---|---|
| calculated: | 70.18 % C, | 6.45 % H, | 3.90 % N, | 10.57 % F; |
| found: | 70.40 % C, | 6.60 % H, | 3.89 % N, | 10.44 % F; |

¹H-NMR (C²HCl₃): δ (2-CH₃) = 1.25 d, J = 7.0 Hz;
δ (NH) = 5.86 bt;
δ (aromat. ortho-CH) = 8.06 d, J = 8.5 Hz.

### Example 4

### Cyclohexylammonium salt of 4-(2,4-difluorobiphenylyl)-2-methyl-4-oxobutanoic acid

4.0 g of 4-(2,4-difluorobiphenylyl)-2-methyl-4-oxobutanoic acid is dissolved in 40 ml acetone and to the clear solution a solution of 1.32 g cyclohexylamine in 20 ml ether is added under constant stirring and cooling at 20°C. Immediately a sediment begins to precipitate and ends after 2 hours of cooling at 0°C. The precipitate is removed and thoroughly washed in cooled acetone. 4.6 g (86.1 % theoret.) of the sought product (melting point 139-140°C) is obtained.

| for C₂₃H₂₇F₂O₃ (403.5) | | | | |
|---|---|---|---|---|
| calculated: | 68.47 % C, | 6.75 % H, | 3.47 % N, | 9.42 % F; |
| found: | 68.18 % C, | 6.74 % H, | 3.42 % N, | 9.25 % F. |

### Example 5

### Potassium salt of 4-(2,4-difluorobiphenylyl)-2-methyl-4-oxobutanoic acid

A solution of 0.5 g KOH in 10 ml methanol is added to a solution of 3.05 g 4-(2,4-difluorobiphenylyl)-2-methyl-4-oxobutanoic acid in 30 ml acetone. The solution is evaporated to an oily residue which gives an amorphous precipitate by mixing in 40 ml ether. The precipitate is dissolved in 60 ml ethanol, the opaque solution is filtered with activated carbon and the clear filtrate is again evaporated. The oily residue is mixed with 40 ml ether and the precipitate is recovered by filtration. 2.35 g (65.3 theoret.) of potassium salt as a monohydrate (melting point 173-174°C) is obtained.

| for C₁₇H₁₃F₂O₃K.H₂O (360.4) | | | |
|---|---|---|---|
| calculated: | 56.65 % C, | 4.19 % H, | 10.54 % F; |
| found: | 56.71 % C, | 4.04 % H, | 10.39 % F. |

### Example 6

### Calcium salt of 4-(2,4-difluorobiphenylyl)-2-methyl-4-oxobutanoic acid

3.05 g of 4-(2,4-difluorobiphenylyl)-2-methyl-4-oxobutanoic acid is dissolved in 55 ml 5N NaOH at 40 °C, the clear solution is heated to 70°C and gradually, a solution of 1.1 g CaCl₂ is added. Upon cooling to 5°C a precipitate is formed which after 2 hours of standing at this temperature is removed and washed on a filter with distilled water until a negative reaction for chloride ions is obtained. The precipitate is then resuspended in 50 ml distilled water, pH is adjusted to 8 with 1N HCl under constant stirring mixed for another 30 minutes and the precipitate is removed. Upon drying to a constant weight 2.7 g (83.6 % theoret.) of calcium salt of 4-(-2,4-difluorobiphenylyl)-2-methyl-4-oxobutanoic acid (melting point 155-157°C) is obtained.

| for C₃₄H₂₆F₄O₆Ca (646.7) | |
|---|---|
| calculated: | 6.18 % Ca; |
| found: | 6.42 Ca. |

### Example 7

### L-lysine salt of 4-(2,4-difluorobiphenylyl)-2-methyl-4-oxobutanoic acid

4.0 g of 4-(2,4-difluorobiphenylyl)-2-methyl-4-oxobutanoic acid is dissolved in 70 ml acetone at 40°C under constant stirring. After cooling to 20°C a solution of 2.15 g L-lysine in 10 ml H₂O is added to the clear solution. The mixture is cooled to 5°C and after stirring for 2 hours at this temperature the precipitate found is removed and washed in 10 ml cooled acetone. Upon drying to a constant weight 3.5 g (59.1 % theoret.) of the product (melting point 173-175°C) is obtained.

| for C₂₃H₂₈N₂F₂O₅ (450.5) | | | | |
|---|---|---|---|---|
| calculated: | 61.32 % C, | 6.26 % H, | 6.22 % N, | 8.43 % F; |
| found: | 61.13 % C, | 6.43 % H, | 6.01 % N, | 8.26 % F. |

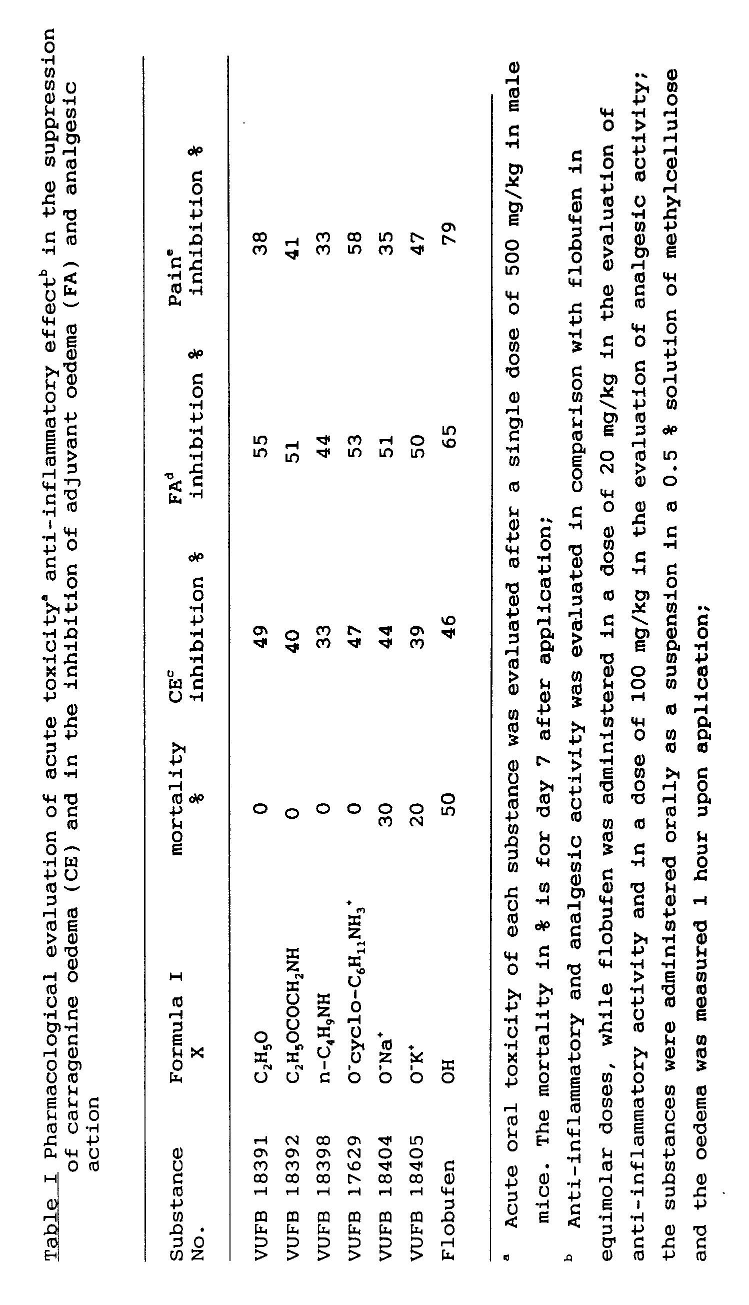

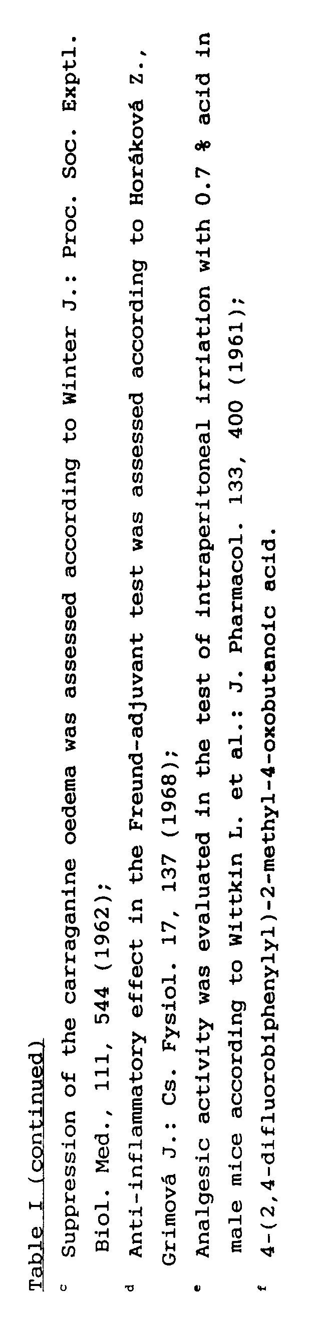

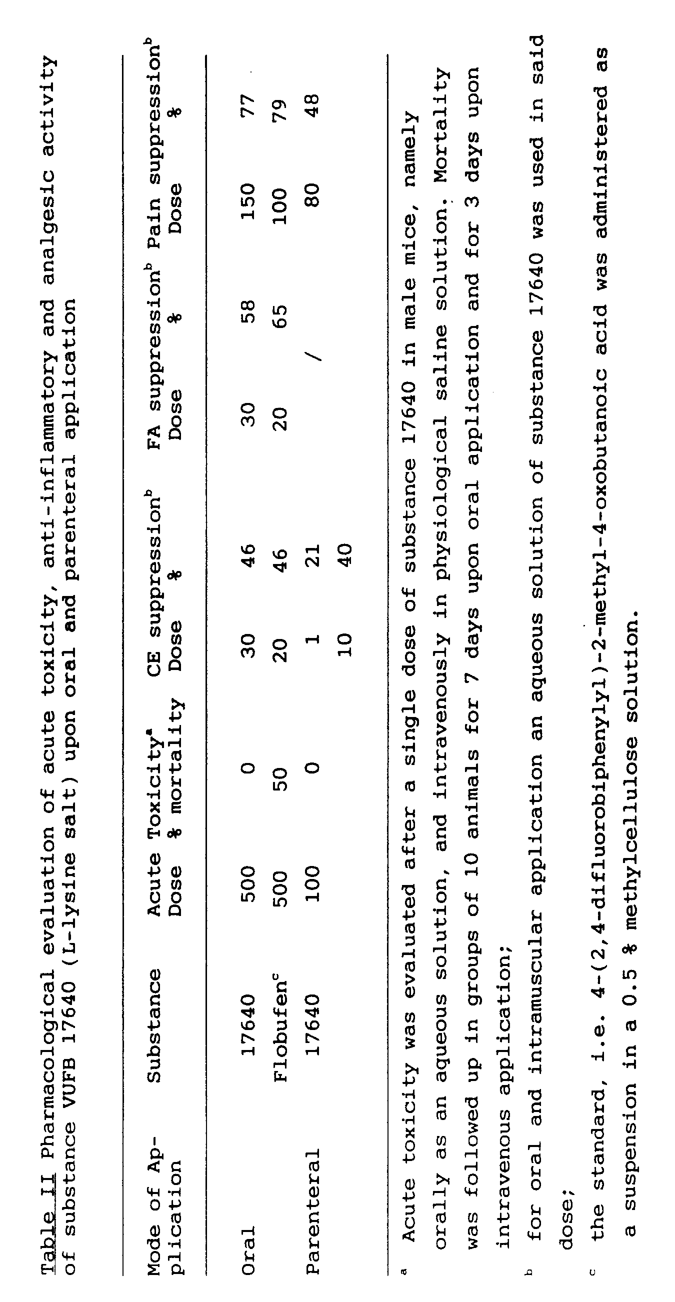

## Claims

1. Functional derivatives of 4-(2,4-difluorobiphenylyl)-2-methyl-4-oxobutanoic acid represented by following general formula (I) 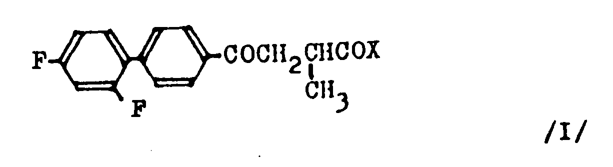 wherein X stands for morpholinyl,
or NHR in which R is an alkyl of 1 to 4 carbon atoms
or a CH₂COOC₂H₅ group,
or OR' in which R' is an alkyl of 1 to 4 carbon atoms,
or O⁻B⁺ group wherein B⁺ is either an alkaline metal cation, alkaline earth cation, cyclohexylamine cation, or lysine cation.

## Patentansprüche

1. Funktionelle Derivate der 4-(2,4-Difluorbiphenylyl)-2-methyl-4-oxobutansäure der allgemeinen Formel 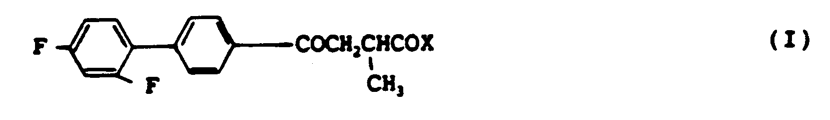 worin X für Morpholinyl oder NHR, wobei R ein C₁₋₄-Alkyl oder eine CH₂COOCH₂H₅-Gruppe bedeutet, oder für OR', worin R' ein C₁₋₄-Alkyl bedeutet, oder für eine Gruppe O⁻B⁺, worin B⁺ entweder ein Alkali- oder Erdalkalimetall-, Cyclohexylamin- oder Lysinkation bedeutet, steht.

## Revendications

1. Dérivés fonctionnels de l'acide 4-(2,4-difluorobiphénylyl)-2-méthyl-4-oxo-butanoïque représentés par la formule générale suivante (I) 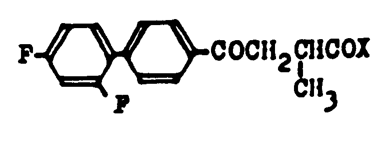 dans laquelle X est une morpholinyle,
ou NHR dans lequel R est un alkyle de 1 à 4 atome(s) de carbone
ou un groupe CH₂COOC₂H₅,
ou OR' dans lequel R' est un alkyle de 1 à 4 atome(s) de carbone,
ou un groupe O⁻B⁺ dans lequel B⁺ est soit un cation de métal alcalin, un cation alcalino-terreux, un cation cyclohexylamine, ou un cation lysine.
